# EUROPEAN PATENT APPLICATION

(11) **EP 2 062 661 A1**
(43) Date of publication of application: **27.05.2009**
(21) Application number: 07117491.6
(22) Date of filing: 28.09.2007
(51) Int. Cl.: B09C 1/10, B09C 1/00, C02F 3/34, C12R 1/01

(54) **Organic de-icer removal**

(71) Applicant: Proviron Holding, 2620 Hemiksem (BE)
(72) Inventor: Cools, Bart, 2860 Sint-katelijne-Waver (BE); Verstraete, Willy, 9032 Wondelgem (BE)
(74) Representative: Luys, Marie-José A.H.

(57) **Abstract**

The present invention relates to a method for the in situ decomposition at a contaminated site, of a compound selected from an organic de-icing and an anti-icing compound or a mixture thereof. Thereto an amount of a biocatalyst is added in-situ to the contaminated site. The biocatalyst is selected from at least one micro-organism or an enzyme present in such a micro-organism or a mixture of two or more of those, wherein the micro-organism comprises a plurality of cells having a membrane containing fatty acids of which at least 20 wt. % are unsaturated fatty acids.

The present invention also relates to a formulation for decomposing a de-icing and/or anti-icing agent, containing an amount of such a biocatalyst.

## Description

The present invention relates to a method for the in situ decomposition at a contaminated site, of an compound selected from an organic de-icing and a freezing point depressing compound or a mixture thereof according to the preamble of the first claim.

### BACKGROUND OF THE INVENTION.

Safety operations at airports during winter include the application of anti- and de-icers to prevent ice formation and remove ice and snow on landing and take-off strips, roads and airplanes. Acetate or formate based de-icers are often used on runways and taxiways, glycol based anti-ice forming compounds are used to prevent ice formation on the airplanes. De-icers and anti-icers can pose a serious threat to receiving waters downstream of airport sites because of the negative impacts on the environment such as high levels of organics, toxicity to aquatic life, oxygen depletion effects,... [1-4]. Although many airports have installed collection systems for collecting anti-icing and de-icing materials at the point of application, anti-icing and de-icing materials used to treat runways and taxiways and those draining from aircrafts as they taxi and take off, are often not collected nor treated.

Because of the considerably high biological oxygen demand (BOD) levels in these liquids, even small amounts of the de-icers can have a serious impact on the receiving environment. A glycol containing de-icing fluid, even when diluted up to 1000-fold, can still have an ultimate biochemical oxygen demand (BODᵤ) in the range of 1 g/L [5]. Glycol concentrations at the airport discharge points may range from very low to 6 g/L and even 24 g/L [3-6]. In creeks adjacent to the airport, the concentration will usually be lower due to dilution, dispersion, and degradation, but may still be up to several hundreds of mg/L [6]. Oxygen depletion in receiving waters due to the degradation of de-icers is however not limited to glycol based de-icers [7]. Typically a mixture of road and wing de-icers accumulates in the sewage system and ditches of the airport site with a typical ratio ranging from 1:10 to 1:1.

Aircraft anti-icing and de-icing chemicals used for wing de-icing are often ethylene or propylene glycol based formulations, containing between 10% and 20% of various additives, including corrosion inhibitors, surfactants, flame retardants, foam suppressants, polymers and even dyes [3, 7]. A class of additives, identified as alkylphenol ethoxylate nonionic surfactants, can be toxic to aquatic organisms and degrade into the known endocrine disrupters nonylphenol and octylphenol [8]. Another class of additives include benzotriazoles. Methylbenzotriazole is used as a corrosion inhibitor and flame retardant in many aircraft anti-icer and de-icer fluids and contributes to the toxicity of these glycol based de-icers [3].

In the last decade, different systems have been developed to remove de-icing compounds in an environmental friendly way. Significant efforts have been made to improve capturing and recovery systems to minimize the amount of de-icers entering the environment, by recovering glycol based de-icers used to anti- and de-ice airplanes at the pollution source [12, 13]. A conventional treatment comprises storage of runoff waters in aerated lagoons before discharge to receiving waters or sewage treatment plants [14]. Other systems include off-site treatment using an aerobic biofilm reactor [5], an aerobic fluidized bed reactor [15], an activated sludge plant [16], an upflow anaerobic sludge blanket reactor or a sequential anaerobic fluidized bed [17] and a sequential biological reactor [18]. Operational difficulties such as sludge bulking and poor settleability were encountered in aerobic activated sludge treatment systems [17]. Further alternative systems include the use of dedicated wetlands where run-off water containing anti- or de-icing compounds is percolated into the soil.

In view of the non-continuous operation of de-icing activities, off-site treatment is expensive. Aerated lagoons or dedicated wetlands require structural adaptations which are not always available at the airport. Moreover, acetate based road de-icers and glycol based de-icers that slough off from airplanes are more difficult to contain and collect and thereby tend to enter the environment in large amounts.

### BRIEF DESCRIPTION OF THE INVENTION.

It is therefore the aim of the present invention to provide a method which permits in situ degradation of the frequently used anti-and de-icers, without requiring substantial structural adaptation of existing constructions.

This is achieved with a method showing the technical features of the characterizing part of the first claim.

Thereto, the method of the present invention is characterized in that an amount of a biocatalyst is added in-situ to the contaminated site, wherein the biocatalyst is selected from at least one micro-organism or an enzyme present in such a micro-organism or a mixture of two or more of those, wherein the micro-organism comprises a plurality of cells containing at least 20 wt. % of unsaturated fatty acids. Preferably at least 20 wt. %, more preferably at least 25 wt. %, most preferably at least 30 wt. % or even at least 40 wt. % of the fatty acids present in the membrane of the micro-organisms are unsaturated fatty acids.

Within the scope of the present invention the unsaturated fatty acids contain between 10 and 20 carbon atoms, preferably between 16 and 20 carbon atoms. Thereby, the fatty acids may be mono-, di- or poly-unsaturated.

The inventor has surprisingly found that micro-organisms in which at least part of the cells have a membrane containing at least 20 wt. % of unsaturated fatty acids, have a higher cell membrane fluidity at the cold temperatures in which the micro-organisms are expected to exert their activity. This way the adaptability to, survival and sustained activity of the micro-organisms at the cold temperatures at which they are used, is ensured and a biocatalyst is provided which is capable of decomposing de-icing and/or anti-icing compounds at the prevailing cold temperatures and where there is a risk that the de- or anti-icing compounds end up in environmental water. This is usually in the temperature region between -5 and 5°C.

According to a preferred embodiment of the present invention, the biocatalyst is selected from at least one micro-organism or at least one enzyme present in the at least one micro-organism capable of decomposing the de-icing and/or anti-icing compound, or a mixture of two or more of them. Thereby, the at least one micro-organism has been selected by growing a micro-organism culture on a medium containing the at least one organic de-icing and/or anti-icing compound and by enriching the at least one micro-organism in aerobic conditions on a medium containing the at least one organic de-icing and/or anti-icing compound at a temperature between -10 and + 10°C. Preferably, the at least one micro-organism has been enriched at a temperature between - 5 and +5°C.

This invention results from the observation by the inventors that there is no need to collect the organic anti-icing or de-icing contaminating compound for further treatment off-site. To the contrary, the inventors have observed that the bio-catalyst of this invention is capable of decomposing the de-icing and/or anti-icing agent in-situ, at the site and in the circumstances where it is used or where it ends up, i.e. on air planes, landing and take-off strips, ditches etc. The bio-catalyst may be applied as such before or after the anti- and/or de-icing agent has been applied, or together with the anti- or de-icing agent.

The inventors have further surprisingly found that when using the biocatalyst of this invention the extent to which decomposition occurs may be increased with a factor 2-10, often 3-5, whereas the decomposition rate can be increased with a factor 3-20 at temperatures prevailing where the use of de- and anti-icing compounds is required, which will usually be between -10 and +10°C. The result is that the present invention permits decomposing the majority of the contaminating compounds applied within the time frame they are left on the surface to which they have been applied, and before they are drawn to and accumulate in run off systems.

In practice the biocatalysts of the present invention has been found to show good activity when applied as such to transportation runways, parking lots, airport runways, taxiways, airplanes etc., or when added to systems wherein the contaminating compound is contained in water for example in sewers, ditches, collection systems, a buffer system, buffer pond, buffer tank, bioreactor or water purification system, even at the low temperatures where anti-icing and de-icing compounds are used, and where micro-organism and enzyme activity is usually negligible.

The in-situ decomposition of anti-icing and de-icing compounds solves the problem of minimizing the COD demand of water receiving bodies adjacent to the places where de- and anti-icing compounds are used, by decomposing the contaminating compound before it contacts receiving water. Conventional de- and anti-agents such as for example glycols, are completely miscible with water and therefore end up easily in receiving water bodies together with the melting water.

Within the framework of the present invention, the in situ decomposition can be carried out within a relatively wide temperature range of between -5 and + 10°C, preferably between 0 and 10 °C, more preferably between 0 and 5°C.

The biocatalyst may be applied in situ as a solid or in a liquid medium. The biocatalyst of this invention may however also be applied together with the anti- and/or de-icing agent.

According to a preferred embodiment of this invention the least one micro-organism is selected to be cold-adapted bacteria from the group of flexibacteraceae, enterobacteriacea, flavobacteria, comamonadaceae or a mixture of two or more of those. The adaptability of these micro-organisms to the cold temperatures in which they are expected to exert their activity seems to be related to the higher cell membrane fluidity which is related to the higher level of unsaturated fatty acids in the membrane.

The biocatalyst of the present invention is suitable for use with a wide variety of commercially available organic de-icing and anti-icing compounds such as acetates, formates for example sodium and potassium formates, glycol, glycerol, mono-ethylene glycol, di-ethylene glycol, monopropylene glycol, dipropylenen glycol or a mixture of two or more of those. Typical examples of acetates include sodium and potassium acetate.

The dose levels with which the biocatalyst is applied in situ may be varied within wide ranges, and will usually be adapted by the person skilled in the art taking into account the amount of organic de-icing and/or anti-icing compound that needs to be decomposed. As such the biocatalyste may be supplied in a concentration of 1- 10⁵ ppm, preferably 1- 10⁴ ppm, more preferably 10 - 5X10³ ppm.

The removal rate can be increased by applying an in situ amount of at least one nutrient which is selected from a compound containing N or P or a mixture of two or more of these compounds. Preferred nutrients include those selected from the group of nitrates and phosphates, for example sodium nitrate, sodium phosphate, ureum. The nutrient is preferably added in an amount such that the ratio COD/N/P is 100 : 1-5: 0.1-2.5.

In practice it has been found that due to the presence of a biocatalyst the decomposition rate constant k at 4°C could be increased with a factor 3-25, often 5-20 and 7-17. In a practical example the decomposition rate could be increased from 0.028/d to 0.204/d and with a factor 17 with addition of NO₃⁻-N and PO₄³⁻-P.

The present invention also relates to a method for applying the biocatalyst of this invention, according to which the biocatalyst is applied together with the de- and/or anti-icing agent.

The present invention further relates to a formulation which contains an amount of an organic anti-icing or de-icing compound or a mixture thereof, which composition is characterized in that it further contains an amount of a biocatalyst selected from at least one micro-organism, at least one enzyme capable of decomposing the de-icing and/or anti-icing compound or a mixture of two or more of them, wherein the micro-organism comprises a plurality of cells having a membrane containing fatty acids. Thereby the biocatalyst is produced using the method described above.

This way fresh biocatalyst is supplied every time de-icing or anti-icing compound is applied, in view of guaranteeing optimum decomposition rates. This also permits adapting the nature and composition of the biocatalyst to the nature of anti- and/or de-icing agent applied. Airports do not necessarily apply the same combination of de- and anti-icing fluids, and do not necessarily use identical application rates. The present invention solves the problem that the nature of the biocatalyst may be adapted to the nature of the fluid applied, as this may vary for different airport areas with anti-icers being predominantly applied at airports sites where aircrafts stand, de-icers being predominantly applied to taxiways and runways, continuous loss of anti-icers from aircrafts taking place at landing and take off strips.

The present invention also relates to a formulation for decomposing a de-icing and/or anti-icing agent, containing an amount of a biocatalyst selected from at least one micro-organism, at least one enzyme capable of decomposing the de-icing and/or anti-icing compound or a mixture of two or more of them, wherein the micro-organism comprises a plurality of cells having a membrane containing fatty acids. Thereby the biocatalyst is produced using the above-described method. Preferably at least part of the fatty acids is unsaturated. Preferably the formulation further contains an amount of at least one nutrient which is selected from a compound containing N or P or a mixture of two or more of these compounds in a ratio such that COD/N/P is 100 : 1-5: 0.1-2.5.

The bio-catalyst or formulation of the present invention may be applied to a surface after it has been contaminated with de-and/or anti-icing agent or in a preventive way in advance of the contamination. For example in winter time de-icing agents are applied to the runway in the evening to prevent ice formation. The biocatalyst can be applied either simultaneously or somewhat after the de-icing agent has been applied.

The present invention also relates to a solid formulation and a method for producing such a solid formulation, according to which the above described formulation is cooled down until solidification is achieved. Thereto usually the formulation needs to be cooled to a temperature of - 20°C or less. At these temperatures the biocatalyst and formulation of this invention may be preserved for a longer period of time, i.e. several months to a year. After thawing, the metabolic activity of the biocatalyst may be restored with at least 75%. Preferably, the biocatalyst of this invention is mixed with an amount of a polyol, more preferably glycerol before cooling to -20°C, to minimize the risk to damaging of the catalyst when thawing.

The biocatalyst or formulation of this invention can be used in any way considered suitable by the person skilled in the art. It may be applied as such to the surface where anti- and/or de-icing agent is to be removed; it may be incorporated in a formulation or form part of the formulation containing the anti- and/or de-icing agent as described above; it may be applied as a dosage to collector systems such as ditches and sewers in which anti- and/or de-icing agents end up when leaving the runways etc. However, numerous other possibilities exist for applying the biocatalyst of this invention.

The invention is further elucidated in the enclosed examples. Herein, the following abbreviations shall mean: BOD, Biological oxygen demand ; BODᵤ, Ultimate biological oxygen demand ; CDW, Cell dry weight ; COD, Chemical Oxygen Demand; COD total total chemical oxygen demand; d day; DO, Dissolved oxygen ; FID, Flame ionization detector ; GC, Gas chromatograph ; IC, Ion chromatograph ; k Biodegradation constant ; MPG, Monopropylene glycol [IUPAC: 1,2-propanediol].

### MATERIAL AND METHODS.

### Enrichment and growth of the enriched microbial consortium.

All chemicals were obtained from Sigma Aldrich (Bornem, Belgium) except when specified.

An enriched microbial consortium for use as biocatalyst was produced by selection for a period of two months at low temperature conditions (4°C) with acetate salt as a sole carbon source, using the procedure described below.

An enrichment medium containing 1.4 g/L CH₃COONa; 143 mg/L NH₄NO₃; 22 mg/L KH₂PO₄ and 10 mg/L Nutriflok 50S (Avecom n.v., Ghent, Belgium) as a nutritive supplement [24] medium was inoculated at 4°C with a microbial culture originating from a soil.

After 3 days of growth at 4°C under continuous agitation (121 rpm), a first microbial consortium was obtained. An aliquot of this first microbial consortium was added to fresh enrichment medium and this was repeated every week for a period of 2 months.

Further enrichment at 4°C was performed in a chemostat (1 L) and the biomass was grown aerobically to a high density (5-10 g CDW/L). The growth medium was dosed continuously and contained 150 g/L CH₃COOH; 21 g/L NH₄NO₃ 3.3 g/L KH₂PO₄ and 1.5 g/L Nutriflok 50S to provide minerals; the pH was maintained at 7.2 with acetic acid from the growth medium. The average removal rate during exponential growth phase was 1.0 g COD/g CDW.d and the average cell yield was 0.25 g CDW/g COD_{removed.} A maximum biomass concentration of 8 g CDW/L was obtained.

### Chemical analyses

Acetate and propionate were analysed using the extraction method for volatile fatty acids in diethyl ether [25]. The samples were analyzed with a capillary FID gas chromatograph 8000 Carlo Erba Instruments (Wigan, UK), containing an Alltech (Deerfield, USA) EC-1000 (30 m, I.D.: 0.32 mm, d_{f}: 0.25 mm) column. The detector and the injector temperature was controlled at 200°C. The column oven started at a temperature of 110°C for 1 minute, thereafter the temperature was increased to 165°C at a rate of 9°C/min. Nitrogen gas was used as the carrier gas at 3 mL/min.

The chemical oxygen demand (COD) analysis was performed using the dichromate method [25]. NO₃⁻-N and PO₄³⁻-P were determined using a Metrohm 761 Compact Ion Chromatograph (IC) equipped with a conductivity detector. The operational parameters were as follows: column metrosep A supp 5, eluens 1.06 g/L Na₂CO₃, flow 0.7 mL/min, sample loop 20 µL. Biomass concentration in cell dry weight (CDW) was determined by analyzing total COD and supernatant COD after 10 minutes centrifugation at 10 000 rpm. By subtracting supernatant COD from total COD, biomass-COD was obtained. To convert biomass-COD into CDW a generally accepted conversion factor of 0.75 was used (biomass-COD * 0.75 = biomass-CDW). The reproducibility of the tests was examined at several occasions and the variability was found to be below 5 to 10% therefore only the mean values are plotted in the figures.

### Batch biodegradation tests.

### Example 1.

In a first test series, 4 test solutions with different acetate and nutrient concentrations were prepared with tap water, as summarized in table 1. For each test solution, the biocatalyst was added in two different concentrations. Thererto, 50 ml of test solution was inoculated with a biocatalyst concentration corresponding to 0.25 and 0.5 g CDW/L test solution respectively. The acetate concentration was measured daily for a period of 8 days, while continuously agitating the mixture (121 rpm) in a temperature controlled room (4 ± 1 °C).

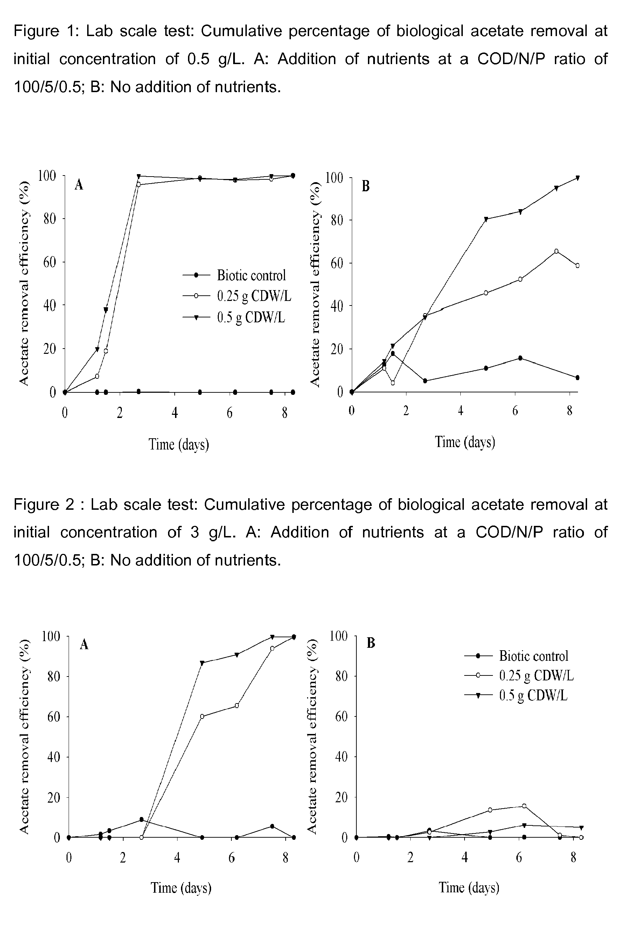

As can be seen from figure 1A, all acetate could be removed within 2.5 days at 4°C at an initial concentration of 0.50 g/L acetate and upon addition of biocatalyst and nutrients. The maximum removal rate was 0.96 g/g CDW.d and 0.51 g/g CDW.d for biomass concentrations of 0.25 and 0.5 g CDW/L, respectively. The mean removal rate was more or less the same, 0.82 and 0.89 g/g CDW.d for 0.25 and 0.5 g CDW/L, respectively.

Without nutrient addition, complete removal of acetate required 8 days with 0.5 g CDW/L and only 60% was removed with 0.25 g CDW/L (Figure 1 B). The mean removal rate was 5 to 7 times lower, i.e. 0.15 and 0.12 g/g CDW.d.

As can be seen from figure 2A, when treating a medium containing 3.0 g/L acetate, higher maximum removal rates of 3.50 and 2.45 g/g CDW.d for 0.25 and 0.5 g CDW/L respectively are observed (Figure 2A). In contrast with the removal of 0.5 g/L acetate, a lag phase of 2.5 days occurred, followed by an exponential acetate removal. Complete removal was obtained after 8 days with nutrient addition.

For each test solution, a control test was performed. For the control, 50 mL of test solution was transferred in a 250 mL flask without biocatalyst addition. No significant acetate breakdown (0-6%) was measured after 8 days (see figure 1A and 2A).

From the above it can be concluded that maximum removal rates for acetate ranged from 0.96 g/g CDW.d up to 3.50 g/g CDW.d at 4°C upon addition of biocatalyst. The initial acetate concentrations ranged from 0.5 up to 3.0 g/L. The latter corresponded to realistic concentrations for dry and wet weather runoff [1,3,6].

### Example 2.

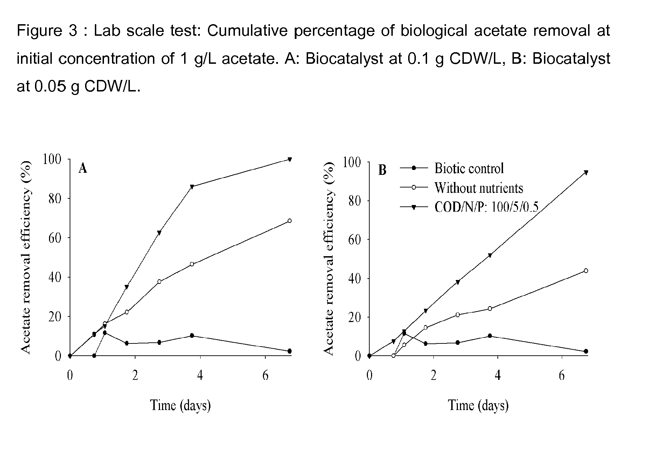

In a second biodegradation test series, the effect of the biocatalyst concentration (0.05 and 0.1 g CDW/L) on the acetate removal capacity was tested (Test 2 in table 1). Thereto the removal rate of 1.0 g/L acetate was tested with 0.1 g CDW/L (Figure 3A) and 0.05 g CDW/L (Figure 3B) of biocatalyst. The medium to be treated was based on surface water to provide low concentrations of N and P. NO₃⁻-N and PO₄³⁻-P concentrations were measured at the end of the test. Samples were taken more frequently at the beginning of the test to evaluate the acetate removal over a period of 7 days.

Within 7 days, 100% acetate was removed with 0.1 g CDW/L and 95% with 0.05 g CDW/L. Under nutrient limited conditions, only 70% and 44% acetate was removed after 7 days with 0.1 and 0.05 g CDW/L respectively. The maximum removal rates were in the same order of magnitude for 0.05 and 0.1 g CDW/L, namely 1.46 g/g CDW.d and 1.43 g/g CDW.d.

With extra mineral nutrients, the maximum removal rates were doubled, namely 3.33 g/g CDW.d and 2.63 g/g CDW.d respectively. After 7 days 7.9 mg/L NO₃⁻-N and 4.4 mg/L PO₄³⁻-P were measured in the control, indicating a low nutrient level of the media. No NO₃⁻-N or PO₄³⁻-P was detected in the treatments without nutrient addition. Nutrient levels at the end of the treatments with extra mineral nutrient addition, were 0.9 mg/L PO₄³⁻-P and 6.9 mg/L NO₃⁻-N for the biocatalyst concentration of 0.1 g CDW/L and 2.3 mg/L PO₄³⁻-P and 7.2 mg/L NO₃⁻-N for 0.05 g CDW/L.

### Example 3.

In a third biodegradation test series, a mixture of acetate and monopropylene glycol was treated with the biocatalyst produced as described above. Monopropylene glycol (MPG) or 1,2-propanediol (C₃H₈O₂) theoretically contains 1.68 g COD/g MPG.

Test media were prepared as presented in Table 1, test 3. The experimental setup was done as in the previous batch tests. Test media were supplemented with 0.02 and 0.15 g CDW/L of biocatalyst respectively. At the beginning of the test period and after 12 days CODₜₒₜₐₗ, acetate, propionate, NO₃⁻-N and PO₄³⁻-P were measured. In addition, the effect of the initial CODₜₒₜₐₗ concentration (800 and 1200 mg COD/L) of a 1:3 COD based mixture of acetate and MPG was tested.

For each test solution, a control test was performed. For the control, 50 mL of test solution was transferred in a 250 mL flask without biocatalyst addition. No significant breakdown (0-6%) was measured after 8 days.

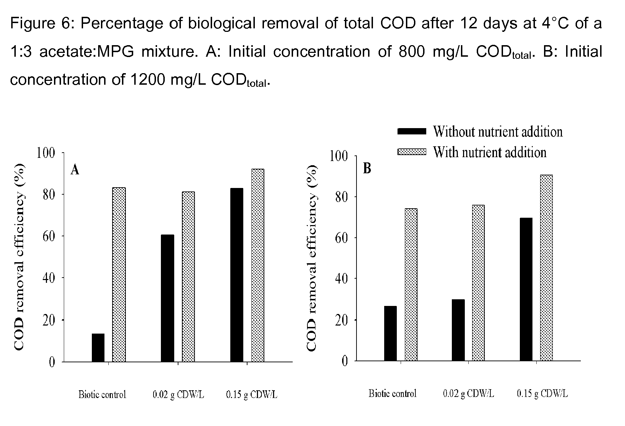

Under nutrient limited conditions, bio-augmentation of the test media with biocatalyst clearly showed an improved removal of CODₜₒₜₐₗ compared to the control (Figure 6). With 0.15 g CDW/L, 83 and 70% of 800 and 1200 mg COD/L was removed in 12 days. In the control, removal percentages were 13 and 26%. The addition of a biomass concentration of 0.02 g CDW/L improved removal to 61% for the lower tested COD concentration (800 mg COD/L). Upon addition of biocatalyst up to 0.15 g CDW/L and nutrients at a COD/N/P of 100/1/0.2, it was observed that 92 and 90% of total COD (MPG and acetate) was removed after 12 days for the initial CODₜₒₜₐₗ of 800 and 1200 mg/L respectively. In the control, microorganisms already present in the surface water, used as medium for the biodegradation test, were capable of degrading MPG in a period of 12 days at 4°C if extra mineral nutrients were supplied. No enhanced removal of total COD was observed with biocatalyst addition up to 0.02 g CDW/L. The same trend was seen for the removal of acetate. When nutrients were provided, 96 to 99% was removed in 12 days, irrespectively of the added biomass concentration. Under nutrient limited conditions, the addition of biocatalyst promoted the removal of acetate up to 73 and 99%. No propionate was detected at the end of the test. After 12 days, no PO₄³⁻-P was detected (detection limit 0.03 mg/L) in the nutrient enriched treatment and a residual of only 1.8 ± 0.1 mg/L NO₃⁻-N and 0.7 ± 0.5 mg/L NO₃⁻-N were measured for the 800 and 1200 mg COD/L set-ups.

### Example 4: Field test.

Two aerated basins with an active volume of 120 m³ were filled with water originating from the ditches (50 ppm COD) at the airport. The products Cryotech E36^{®} (commercially available from Proviron Industries) and Kilfrost ABC-S^{®} (commercially available from Kilfrost) were added to obtain a concentration of 566 mg/L COD for the treatment basin and 500 mg/L COD for the control basin, with a acetate:MPG ratio of 80/20. The biocatalyst was added in such an amount to the first treatment basin that a final biocatalyst concentration of 0.17 kg CDW/m ³ was obtained. The second basin served as a control and no microbial catalyst was added. The external aeration device assured levels of dissolved oxygen of at least 2 mg/L. Samples were taken daily and analyzed for COD.

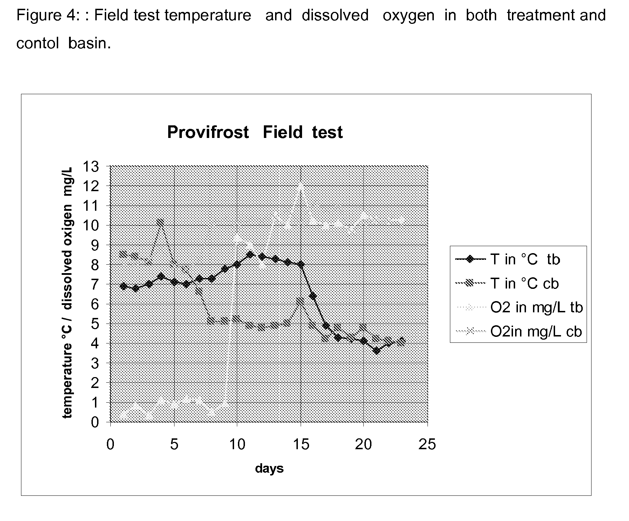

From day 1 to 23 the water temperature declined from 7 to 4°C (Figure 4).Dissolved oxygen in the treatment basin was low during the first 9 days, due to poor aerate. (Figure 4). From day 9 dissolved oxygen was increased to a level of 10 mg/L. Because of the oxygen depletion during the first 9 days, removal of COD in the treatment was slow (from 560mg/L to 520 mg/L in 7 days).

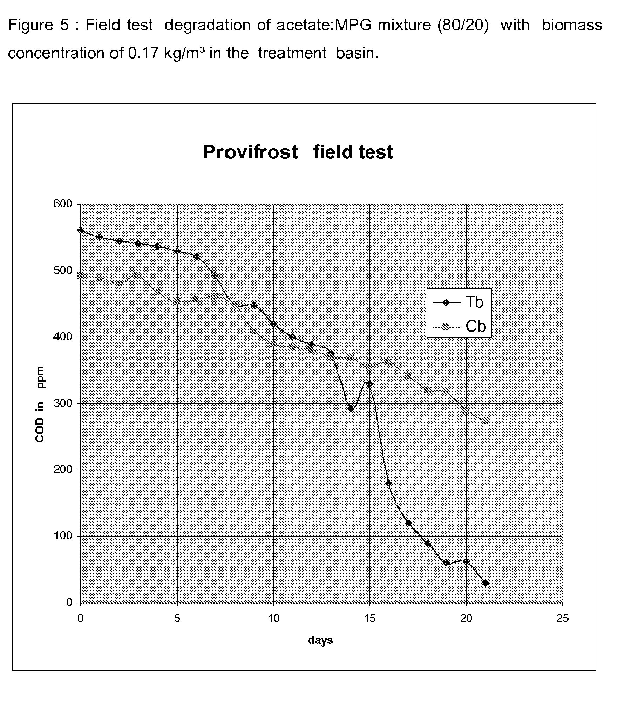

From day 7 on, a linear removal of COD in the treatment basin was observed (figure 5). Complete removal of COD in the treatment basin was obtained in 18 days. In the control basin COD removal was poor 3 times slower as in the treatment basin. The pH (6.5 ± 0.2) in the treatment basin was not significantly different from the control. 2kg Urea is added to the treatment basin after 10 days in order to control the COD/N/P balance.

**Table 1 : Test media for the batch degradation tests of acetate (test 1 and 2) and a 1:3 COD based acetate:MPG mixture (test 3) with biomass concentrations ranging from 0.02 to 0.5 g CDW/L. (NA: not applicable; NM: not measured)**

| | mg/L CODₜₒₜₐₗ * | CH₃COOH ** | NH₄NO₃-N | KNO₃-N (measured) | KH₂PO₄-P (measured) | Nutriflok 50S |
|---|---|---|---|---|---|---|
| Test 1 | | | | | | |
| 1 | NM | 503 ± 35 | | | | |
| 2 | NM | 417 ± 59 | 25 | NA | 2.5 | 2.5 |
| 3 | NM | 2871 ± 44 3037 ± | | | | |
| 4 | NM | 111 | 150 | NA | 15 | 15 |
| | | | | | | |
| Test 2 | | | | | | |
| Control medium | NM | 1111 | NA | 50 | 5 | 0 |
| Without nutrients | NM | 1139±60 | | | | |
| With nutrients | NM | 1164 ± 58 | NA | 50 | 5 | 0 |
| | | | | | | |
| Test 3 | | | | | | |
| | 780 ± | | | | | |
| 1 | 142 | 199 ± 3 | | | | |
| | 792 ± | | | 8.0 | 0.2 | |
| 2 | 105 | 207 ± 3 | NA | (9.0 ± 2.1) | (1.4 ± 0.3) | 0 |
| 3 | 1139 ± | 347 ± 11 | | | | |
| | 141 | | | | | |
| 4 | 1273 ± | 341 ± 28 | NA | 12.0 | 2.4 | 0 |
| | 280 | | | (16.7 ± 2.2) | (2.6 ± 0.5) | |

| | | | | | | |
|---|---|---|---|---|---|---|
| *Dosed as CH₃COONa and 1,2-propanediol - measured in triplicate as total COD **Dosed as CH₃COONa - measured in triplicate as CH₃COOH | | | | | | |

### REFERENCES

[1] S. R. Corsi, N. L. Booth, D. W. Hall, Aircraft and runway deicers at general Mitchell international airport, Milwaukee, Winsconsin, USA. 1. Biochemical oxygen demand and dissolved oxygen in receiving streams, Environ. Toxicol. Chem. 2001, 20 (7), 1474-1482.
[2] S. I. Hartwell, D. M. Jordahl, J. E. Evans, E.B. May, Toxicity of aircraft de-icer and anti-icer solutions to aquatic organisms, Environ. Toxicol. Chem. 1995, 14 (8), 1375-1386.
[3] S. R. Corsi, S. W. Geis, J. E. Loyo-Rosales, C. P. Rice, R. J. Sheesley, G. G. Failey, D. A. Cancilla, Characterization of aircraft deicer and anti-icer components and toxicity in airport snowbanks and snowmelt runoff, Environ. Sci. Technol. 2006, 40, 3195-3202.
[4] S. R. Corsi, D. W. Hall, S. W. Geis, Aircraft and runway deicers at general Michell international airport, Milwakee, Wisconsin, USA. 2. Toxicity of aircraft and runway deicers, Environ. Toxicol. Chem. 2001, 20 (7), 1483-1490.
[5] S. I. Safferman, R. A. Azar, S. Sigler, Passive nutrient addition for the biodegradation of ethylene glycol in storm water, J. Environ. Sci. Health. Part A 2002, 37 (5), 955-967.
[6] S. R. Corsi, G. R. Harwell, S. W. Geis, D. Bergman, Impacts of aircraft de-icer and anti-icer runoff on receiving waters from Dallas/Fort Worth International Airport, Texas, USA, Environ. Toxicol. Chem. 2006, 25 (11), 2890-2900.
[7] D. M. Ramakrishna, T. Viraraghavan, Environmental impact of chemical deicers - a review, Water, Air, Soil Pollut. 2005, 166, 49-63.
[8] S. R. Corsi, D. H. Zitomer, J. A. Field, D. A. Cancilla, Nonylphenol ethoxylates and other additives in aircraft deicers, antiicers, and waters receiving airport runoff, Environ. Sci. Technol. 2003, 37 (18), 4031-4037.
[9] C. A. Staples, J. B. Williams, G. R. Craig, K. M. Roberts, Fate, effects and potential environmental risks of ethylene glycol: a review, Chemosphere 2001, 43, 377-383.
[10] J. S. LaKind, E. A. McKenna, R. P. Hubner, R. G. Tardiff, A review of the comparative mammalian toxicity of ethylene glycol and propylene glycol, Crit. Rev. Toxicol. 1999, 29 (4), 331-365.
[11] S. S. Bang, D. Johnston, Environmental effects of sodium acetate/formate deicer Ice Shear™, Arch. Environ. Contam. Toxicol. 1998, 35 (4), 580-587.
[12] Storm Water Technology Fact Sheet: Airplane deicing fluid recovery systems (Ed: US Environmental Protection Agency Office of Water), EPA Publication EPA/832/F-99/043, Washington D.C. (USA), 1999**.**
[13] D. Mericas, Advances in airport stormwater management, Water Environmental and Technology 2005, 17 (1), 40-45.
[14] S. Chong, H. Garelick, D. M. Revitt, R. B. E. Shutes, P. Warrall, D. Brewer, The microbiology associated with glycol removal in constructed wetlands, Water Sci. Technol. 1999, 40 (3), 99-107.
[15] S. I. Safferman, G. S. Siruvalure, L. E. Foppe, Deicing fluid treatment in batch-loaded aerobic fluidized bed reactor, J. Environ. Eng. -ASCE 1998, 124 (1), 11-15.
[16] A. C. Kilroy, N. F. Gray, Pilot plant investigation of the treatability of ethylene glycol by activated sludge, Environ. Technol. 1992, 13, 293-300.
[17] P. T. Tham, K. J. Kennedy, Anaerobic biodegradation of aircraft deicing fluid in UASB reactors, Water Res. 2004, 38, 2515-2528.
[18] Y. Saheb, K. S. Narasiah, Degradation rate of aircraft deicing fluid in a sequential biological reactor, Water Sci. Technol. 1992, 26 (9-11), 2061-2064.
[19] S. Castro, L. C. Davis, L. E. Erickson, Natural, cost-effective, and sustainable alternatives for treatment of aircraft deicing fluid waste, Environ. Prog. 2005, 24 (1),26-33.
[20] M. S. Switzenbaum, S. Veltman, D. Mericas, B. Wagoner, T. Schoenber, Best management practices for airport deicing stormwater, Chemosphere 2001, 43, 1051-1062.
[21] C. McGahey, J. Bower, Biodegradation of ethylene glycol in simulated subsurface environments, Water Sci. Technol. 1992, 26, 41-49.
[22] E. Jungo, P. Schöb, Disposal of de-icing effluents by irrigation. Accepted paper for the 3rd IWA Leading-Edge Conference and Exhibition on Waster and Wastewater Treatment Technologies, 6-8 June 2005, Sapporo, Japan, 2005.
[23] R. J. Rice, T. A. Anderson, J. R. Coats, Evaluation of the use of vegetations for reducing the environmental impact of deicing agents, Phytoremediation of Soil and Water Contaminants ACS Symposium Series 1997, 664, 162-176.
[24] S. Vansever, P. Bossier, A. Vanderhasselt, M. Beeckman, J. Van Der Zanden, D. Weytjens, C. Mingneau, W. Verstraete, Improvement of activated sludge performance by the addition of Nutriflok 50 S, Water Res. 1997, 31 (2), 366-371.
[25] Standard Methods for the Examination of Water and Wastewater, 18th ed. (Ed: APHA/AWWA/WEF), Washington DC, USA, 1992**.**
[26] W. Verstraete, E. Van Vaerenbergh, Chapter 2. Aerobic activated sludge, in Biotechnology (Eds: H. J. Rehm, G. Reed), Vol. 8, Wiley-VCH, Weinheim 1986, 44-112.
[27] D. M. Revitt, P. Worrall, Low temperature biodegradation of airport de-icing fluids, Water Sci. Technol. 2003, 48 (9), 103-111.
[28] H. K. French, S. E. A. T. M. Van der Zee, A. Leijnse, Transport and degradation of propyleneglycol and potassium acetate in the unsaturated zone, Contam. Hydrol. 2001, 49, 23-48.
[29] D. P. Shupack, T. A. Anderson, Mineralization of propylene glycol in root zone soil, Water, Air, Soil Pollut. 2000, 118, 53-64.
[30] G. M. Klecka, C. L. Carpenter, B. D. Landenberger, Biodegradation of aircraft deicing fluids in soil at low temperatures, Ecotoxicol. Environ. Saf. 1993, 25, 280-295.
[31] S. Veltman, T. Schoenberg, M. S. Switzenbaum, Alcohol and acid formation during the anaerobic decomposition of propylene glycol under methanogenic conditions, Biodegradation 1998, 9 (2), 113-118.
[32] P. Jaesche, K. U. Totsche, I. Kögel-Knabner, Transport and anaerobic biodegradation of propylene glycol in gravel-rich soil materials, J. Contam. Hydrol. 2006, 85, 271-286.

## Claims

1. A method for the in situ decomposition at a contaminated site, of a compound selected from an organic de-icing and an anti-icing compound or a mixture thereof, **characterized in that** an amount of a biocatalyst is added in-situ to the contaminated site, wherein the biocatalyst is selected from at least one micro-organism or an enzyme present in such a micro-organism or a mixture of two or more of those, wherein the micro-organism comprises a plurality of cells having a membrane containing fatty acids of which at least 20 wt. % are unsaturated fatty acids.

2. A method as claimed in claim 1, **characterized in that** at least 25 wt. %, preferably at least 30 wt. %, more preferably at least 40 wt. % of the fatty acids are unsaturated.

3. A method as claimed in claim 1 or 2, **characterized in that** the unsaturated fatty acids contain between 10 and 20 carbon atoms, preferably between 16 and 20 carbon atoms.

4. A method as claimed in any one of claims 1-3, **characterized in that** the biocatalyst is selected from at least one micro-organism or at least one enzyme present in the at least one micro-organism capable of decomposing the de-icing and/or anti-icing compound, or a mixture of two or more of them, wherein the at least one micro-organism has been selected by growing a micro-organism culture on a medium containing the at least one organic de-icing and/or anti-icing compound and by enriching the at least one micro-organism in aerobic conditions on a medium containing the at least one organic de-icing and/or anti-icing compound at a temperature between -10 and + 10°C.

5. A method as claimed in claim 4, **characterized in that** the at least one micro-organism has been enriched at a temperature between -5 and 5 °C.

6. A method as claimed in any one of claims 1-5, **characterized that** the in situ decomposition is carried out at a temperature of between -5 and + 10°C, preferably between -5 and + 5 °C.

7. A method as claimed in any one of claims 1-6, **characterized in that** the least one micro-organism selected from the group of flexibacteraceae, enterobacteriacea, flavobacteria, comamonadaceae or a mixture of two or more of those.

8. A method as claimed in any one of claims 1-7, **characterized in that** the biocatalyst is applied in situ as a solid or in a liquid medium.

9. A method as claimed in any one of claims 1-8, **characterized in that** the at least one de-icing and/or anti-icing compound is selected from the group of an acetate, a formate, glycol, mono-ethylene glycol, di-ethylene glycol, monopropylene glycol, dipropylenen glycol or a mixture of two or more of those.

10. A method as claimed in any one of claims 1-9, **characterized in that** the biocatalyst is added in a concentration of 1- 10⁵ ppm, preferably 1- 10⁴ ppm, more preferably 10 - 5X10³ ppm.

11. A method as claimed in any one of claims 1-10, **characterized in that** in situ an amount is applied of at least one nutrient which is selected from a compound containing N or P or a mixture of two or more of these compounds.

12. A method as claimed in claim 11, **characterized in that** the N or P containing nutrient is selected from the group of nitrates and phosphates.

13. A method as claimed in claim 11 or 12, **characterized in that** the nutrient is added in an amount such that the ratio COD/N/P is 100 : 1-5: 0.1-2.5.

14. A formulation containing an amount of an organic anti-icing or de-icing compound or a mixture thereof, **characterized in that** the composition further contains an amount of a biocatalyst selected from at least one micro-organism, at least one enzyme capable of decomposing the de-icing and/or anti-icing compound or a mixture of two or more of them, wherein the micro-organism comprises a plurality of cells having a membrane which is enriched in unsaturated fatty acids and which contains at least 20 wt. % of unsaturated fatty acids.

15. A formulation as claimed in claim 14, **characterized in that** the biocatalyst has been obtained according to the method of any one of claims 1-13.

16. A formulation as claimed in claim 14 or 15, **characterized in that** the at least one micro-organism is obtained by selection form a micro-organism culture grown on a medium containing the at least one organic de-icing and/or anti-icing compound and enriched in aerobic conditions on a medium containing the at least one organic de-icing and/or anti-icing compound at a temperature between -10 and + 25°C.

17. A formulation for decomposing a de-icing and/or anti-icing agent, containing an amount of a biocatalyst selected from at least one micro-organism, at least one enzyme capable of decomposing the de-icing and/or anti-icing compound or a mixture of two or more of them, wherein the micro-organism comprises a plurality of cells having a membrane which is enriched in unsaturated fatty acids and which contains at least 20 wt. % of unsaturated fatty acids.

18. A formulation as claimed in claim 17 **characterised in that** the formulation further contains an amount of at least one nutrient which is selected from a compound containing N or P or a mixture of two or more of these compounds in a ratio such that COD/N/P is 100 : 1-5: 0.1-2.5.

19. A process for producing a solid formulation containing the biocatalyst obtained with the method of any one of claims 1-13, or the formulation of claim 17 or 18, **characterized in that** the biocatalyst is cooled to a temperature of at least -20°C until solidification is obtained and the biocatalyst is stored at that tempterature.

20. A process as claimed in claim 19, **characterized in that** the biocatalyst is first mixed with an amount of a polyol, preferably glycerol before cooling to -20°C.
